# EUROPÄISCHE PATENTSCHRIFT

(11) **EP 1 531 744 B1**
(45) Veröffentlichungstag und Bekanntmachung des Hinweises auf die Patenterteilung: **14.10.2009**
(21) Anmeldenummer: 03790750.8
(22) Anmeldetag: 25.08.2003
(51) Int. Cl.: A61B 17/16

(54) **VORRICHTUNG ZUR BEARBEITUNG VON TEILEN**
DEVICE FOR PROCESSING PARTS
DISPOSITIF POUR TRAITER DES PARTIES

(30) Priorität: 26.08.2002 DE 10239673
(43) Veröffentlichungstag der Anmeldung: 25.05.2005
(73) Patentinhaber: Schwarz, Markus, 82067 Ebenhausen (DE); Pott, Peter, 68309 Mannheim (DE)
(72) Erfinder: Schwarz, Markus, 82067 Ebenhausen (DE); Pott, Peter, 68309 Mannheim (DE)
(74) Vertreter: Naumann, Ulrich
(86) Internationale Anmeldenummer: PCT/DE2003/002846
(87) Internationale Veröffentlichungsnummer: WO 2004/019785

(56) Entgegenhaltungen:
- EP-A- 0 456 103
- WO-A-97/30826
- DE-A- 19 700 402
- D'ATTANASIO S ET AL: "A semi-automatic handheld mechatronic endoscope with collision-avoidance capabilities" PROC.2000 IEEE INT.CONF.ON ROBOTICS AND AUTOMATION, April 2000 (2000-04), Seiten 1586-1591, XP002270615 PISCATAWAY,NJ,USA ISBN: 0-7803-5886-4
- JAKOPEC M ET AL: "Acrobot: a hands-on robot for total knee replacement surgery" PROC.7TH INT.WORKSHOP ON ADVANCED MOTION CONTROL, MARIBOR,SLOVENIA,3-5 JULY 2002, 3. Juli 2002 (2002-07-03), Seiten 116-120, XP010598955 IEEE,PISCATAWAY,NJ,USA
- WEI TECH ANG ET AL: "Design and implementation of active error canceling in hand-held microsurgical instrument" PROC. 2001 IEEE RSJ INT.CONF. ON INTELLIGENT ROBOTS AND SYSTEMS 29 OCT.-03 NOV., 2001, Seiten 1106-1111, XP002270616 PISCATAWAY,NJ,USA ISBN: 0-7803-6612-3

## Beschreibung

Die Erfindung betrifft eine Vorrichtung zur Bearbeitung von Teilen jedweder Art, insbesondere von Knochen, Organen etc. des menschlichen/tierischen Körpers, mit einem Gehäuse, einem dem Gehäuse zugeordneten Werkzeug und einer eine Relativbewegung zwischen dem Gehäuse und dem Werkzeug bewirkenden Betätigungseinheit.

Zur operativen Manipulation von Knochen sind insbesondere in der orthopädischen Chirurgie bereits passive Navigatoren bekannt, welche den Anwender bei der Orientierung des Werkzeugs am Patienten und bei der präzisen operativen Planung der Operation unterstützen. Alle Arbeitsschritte werden jedoch vom Anwender selbst ausgeführt.

Nachteilig bei diesen Navigationssystemen ist, dass computernavigierte Werkzeuge nicht automatisch die gewünschte Position erreichen, da Muskeltremor und unwillkürliche transiente Bewegungen des Operateurs während des Arbeitsgangs dem entgegenstehen. Die präoperative Planung wird entsprechend fehlerbehaftet umgesetzt.

Insbesondere in der Hüft- und Knieendoprothetik sowie bei der Revisionsoperation des Hüftgelenkes und beim Ersatz des vorderen Kreuzbandes ist der Einsatz von Robotern bekannt, welche am Patienten als aktive Navigatoren selbständig Operationsschritte ausführen. Diese können vorher an einer Workstation oder direkt im Operationssaal programmiert werden.

Ein Nachteil derartiger Systeme ist zum einen die verlängerte Operationsdauer, zum anderen die sehr hohen Anschaffungs- und Unterhaltskosten sowie der zusätzliche Platzbedarf derartiger Systeme. Darüber hinaus fehlt vielen Chirurgen beim Einsatz eines Roboters subjektiv die Prozesskontrolle über das Geschehen auf dem OPTisch.

Aus der WO-A-9730826 ist für sich gesehen eine robotische Positioniervorrichtung zur Positionierung eines Positionierkopfes in Bezug auf ein zu bearbeitendes Werkstück bekannt. Die Vorrichtung umfasst einen Positionierkörper sowie ein Lokalisierungsmesssystem zur Bestimmung der Raumkoordinaten eines zu bearbeitenden Werkstücks einerseits und des Positionierkopfes andererseits. Mit dem Lokalisierungsmesssystem kommuniziert ein Steuerdatensystem für eine Positioniersteuereinheit, mit deren Hilfe der Positionierkopf in eine vorgebbare Lage in Bezug auf das zu bearbeitende Werkstück verbracht werden kann.

Aus dem Dokument "A semi-automatic handheld mechatronic endoscope with collision-avoidance capabilities", von S. D'Attanasio et al., Proceedings of the 2000 IEEE International Conference on Robotics & Automation, San Francisco, CA, April 2000 ist ein semi-automatisches mechatronisches Werkzeug für die computerunterstützte Endoskopie bekannt. Das Endoskop umfasst eine lenkbare Spitze, die mittels eines an einem Handgriff des Endoskops angeordneten Hebels in einem Freiheitsgrad manuell steuerbar ist. Mittels einer Einrichtung zur Kollisionsverhinderung wird ein Kontakt der Spitze mit sensiblen anatomischen Regionen vermieden.

Aus der EP-A-0 456 103 ist für sich gesehen ein bildgeführtes Robotersystem für die genaue Chirurgie bekannt.

Eine Vorrichtung der gattungsbildenden Art ist bereits aus der DE 197 00 402 C2 bekannt. Das dort beschriebene Instrument ermöglicht es, das unwillkürlich auftretende Handzittern (Tremor) bei manuellen Arbeiten an feinen Strukturen weitestgehend zu kompensieren. Diese Kompensation des Muskeltremors ist insbesondere in der Mikrochirurgie von großer Bedeutung. Dabei sind an dem Instrument Beschleunigungs- und Winkelgeschwindigkeitssensoren angebracht, die ein mit der Bewegung des Instruments korreliertes mechanisches oder elektrisches Signal liefern. Diese Sensorsignale werden zunächst verstärkt und sodann hinsichtlich der Frequenz, der Amplitude und der Richtung bzw. Beschleunigung des Werkzeugs analysiert. Auf diese Weise können die unerwünschten Bewegungen evaluiert und von beabsichtigten Bewegungen unterschieden werden. Anhand der Daten können die Aktoren so angesteuert werden, dass diese zur Kompensation der unerwünschten Auslenkung des handgehaltenen Abschnitts Relativbewegungen des beweglichen Abschnitts bewirken.

Bei der bekannten Vorrichtung ist jedoch problematisch, dass zwar der Muskeltremor weitestgehend aus der Werkzeugbewegung herausgehalten wird, dass jedoch die Positionierung des Werkzeugs nicht programmierbar gesteuert werden kann, sondern manuell durchgeführt werden muss. Wenn ein Chirurg das Bearbeitungswerkzeug, beispielsweise einen Bohrer, an einer falschen Stelle oder in einer falschen Winkelstellung ansetzt, wird dieser Fehler von der bekannten Vorrichtung nicht erkannt und folglich auch nicht korrigiert.

Der vorliegenden Erfindung liegt nunmehr die Aufgabe zugrunde, eine Vorrichtung zur Bearbeitung von Teilen jedweder Art, insbesondere von Knochen, Organen etc. des menschlichen/tierischen Körpers, der eingangs genannten Art anzugeben, welche die Präzision einer robotischen Bearbeitung mit dem subjektiven Gefühl von Prozesskontrolle, das ein Chirurg mit einem handgehaltenen Gerät bekommt, kombiniert.

Erfindungsgemäß wird die voranstehende Aufgabe durch die Merkmale des Patentanspruchs 1 gelöst. Danach ist die in Rede stehende Vorrichtung derart ausgebildet, dass die Position des Werkzeugs detektierbar und die Position des zu bearbeitenden Teils detektierbar und/oder vorgebbar ist, wobei die Betätigungseinheit so ansteuerbar ist, dass sie das Werkzeug innerhalb eines vorgegebenen Arbeitsbereichs in eine vorgebbare Relativlage gegenüber dem zu bearbeitenden Teil verbringt, wobei eine Reglereinheit zur Auswertung der Positionsdaten und zur Generierung der Steuergrößen für die Betätigungseinheit vorgesehen ist.

In erfindungsgemäßer Weise ist zunächst erkannt worden, dass auch mit einem handgehaltenen Gerät trotz des Muskeltremors und anderer unvermeidbarer Fehlbewegungen des Anwenders eine maschinelle Präzision bei der Bearbeitung von Teilen jedweder Art, insbesondere bei mirkrochirurgischen Operationen, erreichbar ist, wie sie von Verfahren, bei denen Roboter als aktive bzw. semiaktive Navigatoren eingesetzt werden, bereits bekannt ist.

Dazu werden erfindungsgemäß zunächst die Positionen des Werkzeugs und des zu bearbeitenden Teils detektiert. Die detektierten Positionsdaten werden sodann mit vorgegebenen Positionsdaten verglichen. Diese Auswertung wird von einer Reglereinheit durchgeführt, die anhand der gemessenen Abweichung sodann die Steuergrößen für eine Betätigungseinheit generiert, welche das Werkzeug in eine vorgebbare Relativlage gegenüber dem zu bearbeitenden Teil verbringt. Die Betätigungseinheit bewirkt dabei eine Relativbewegung des Werkzeugs gegenüber einem von dem Anwender gehaltenen Gehäuse. Die durch den Bearbeitungsvorgang aufgrund mechanischer Rückkopplung zwischen dem Werkzeug und der Hand des Anwenders verursachten Reaktionsbewegungen des Anwenders werden während des gesamten Bearbeitungsvorgangs korrigiert. Durch die handgehaltene Anwendung wird dem Operateur eine höhere Prozesskontrolle als bei den bekannten Robotersystemen verschafft.

In vorteilhafter Weise ist die Reglereinheit als eine adaptive Reglereinheit ausgeführt.

Die beiden Prozesse der Detektion der Positionen von Werkzeug und zu bearbeitendem Teil sowie des Ansteuems der Betätigungseinheit werden in bevorzugter Weise kontinuierlich oder repetitiv durchgeführt. Somit ist es möglich, auch schnelle bzw. hochfrequente Bewegungen des Werkzeugs - z. B. verursacht durch den Muskeltremor des Chirurgen - sowie Bewegungen des zu bearbeitenden Teils - beispielsweise Zittern des Patienten - zu erfassen und entsprechend zu korrigieren.

Das Werkzeug kann als in allen sechs Freiheitsgraden gegenüber dem Gehäuse bewegbar ausgestaltet sein. Die Freiheitsgrade setzen sich aus Verschiebungen entlang der drei kartesischen Achsen (x, y, z) zusammen, wobei eine Verschiebung entlang der z-Achse den Vorschub des Werkzeugs darstellt. Zum Ausgleich von Winkelfehlstellungen des Werkzeugs könnten auch der Steigungswinkel α und der Gierwinkel ψ gegenüber dem Gehäuse veränderbar sein. Der sechste Freiheitsgrad ist die Rotation des Werkzeugs um die z-Achse, was der eigentlichen Bohrerdrehung entspricht.

Das Gehäuse, das während der Bearbeitung von dem Anwender in der Hand gehalten wird, könnte als Handgriff, Pistolengriff oder ähnlichem ausgebildet sein. Durch diese Ausgestaltung ergibt sich eine gute Handhabbarkeit des Geräts, was für den Anwender ein Höchstmaß an Kontrolle über den Arbeitsprozess bedeutet.

Zur Erfassung der Positionsdaten des Werkzeugs und des zu bearbeitenden Teils könnte ein externes Positionserfassungssystem vorgesehen sein, welches in vorteilhafter Weise mit optischen und/oder akustischen, magnetischen, mechanischen oder radioaktiven Signalen arbeiten könnte. Aus der Anmeldung DE 102 25 077.4, die am Anmeldetag der vorliegenden Erfindung noch nicht offen liegt, ist bezüglich optischer Trackingsysteme bereits Folgendes bekannt:

Durch die Verwendung von mehr als zwei optischen Sensoren kann Redundanz in Trackingsystemen eingeführt werden, die unter anderem - aber nicht nur - zur Erhöhung der Trackinggenauigkeit, -geschwindigkeit und -robustheit verwendbar ist. So kann beispielsweise ein bewegter Sensor unter Verwendung der Informationen von momentan unbewegten Sensoren kalibriert werden.

Weiterhin erlaubt die Verwendung von passiven oder aktiven Farbmarkierungen auf den zu trackenden Objekten die Differenzierung der verschiedenen Marker, was unter anderem zu einer Erhöhung der Trackinggeschwindigkeit führt.

Die beim optischen Tracking anfallende Bildverarbeitung lässt sich durch die Verwendung spezieller Hardware, mit beispielsweise FPGAs (Field programmable Gate Arrays), deutlich beschleunigen. In einer bevorzugten Variante eines solchen Aufbaus kann jedem Bildsensor eine entsprechende Hardware zugeordnet sein. Ein unter Umständen vorhandenes Host- oder Steuersystem wird durch die Verwendung von beispielsweise FPGA Hardware zum Teil oder vollständig entlastet.

Dem Anwender steht somit ein großes, flexibles und unverschattetes Arbeitsfeld zu Verfügung, wodurch ein Arbeiten ohne Änderung von Operationsumfeld und Operationsablauf ermöglicht wird.

Zur leichteren Identifizierung der zu verfolgenden Objekte - Werkzeug und zu bearbeitendes Teil - könnten diese in vorteilhafter Weise mit Markierungen versehen sein, die von dem Trackingsystem detektiert werden. Im Rahmen einer OP-Planung kann die aktuelle Lage des an dem zu bearbeitenden Knochen angeordneten Markers mit einem präoperativen CT des Patienten abgeglichen werden. Dem Werkzeug könnten dabei mindestens drei voneinander beabstandete Marker zugeordnet sein, wodurch nicht nur die Position zum Beispiel der Werkzeugspitze detektierbar sondern auch die Orientierung des Werkzeugs im Raum bestimmbar ist.

Da ein an der Werkzeugspitze angebrachter Marker nach Eindringen des Werkzeugs in das zu bearbeitende Teil optisch nicht mehr zugänglich ist, könnte in besonders vorteilhafter Weise dem Gehäuse ein detektierbarer Marker zugeordnet sein. Weiterhin könnte dann ein dem Gehäuse und/oder dem Werkzeug mittelbar oder unmittelbar zugeordneter innerer Sensor vorgesehen sein, der zur Bestimmung der Relativlage zwischen dem Gehäuse und dem Werkzeug dient. In bevorzugter Weise könnten hierfür mechanische Weggeber oder Winkelsensoren zum Einsatz kommen. Durch diese indirekte Positionsbestimmung des Werkzeugs ist die Funktionsweise der Vorrichtung während des gesamten Arbeitsvorgangs, insbesondere also auch nach Eindringen der Werkzeugspitze in das zu bearbeitende Teil, gewährleistet.

Zur Positionierung des Werkzeugs könnte die Betätigungseinheit Aktoren umfassen, welche in dem vorgegebenen Arbeitsbereich des Werkzeugs in jeder Raumrichtung statische und/oder dynamische Kräfte aufbringen könnten. Mittels der Aktoren sollen Fehlpositionierungen des Werkzeugs durch den Operateur sowie Lageabweichungen während des Bearbeitungsvorganges (z. B. "Verlaufen" des Bohrers im Knochen) korrigiert werden. Es wäre denkbar, alle auftretenden Kräfte und Momente während des gesamten Arbeitsganges zu überwachen. Auf diese Weise könnten dann z. B. auch ruckartig auftretende Bewegungen mit großer Amplitude ("Verreißen") registriert werden, wobei sich das Werkzeug beim Auftreten einer derartigen Bewegung automatisch abschalten könnte.

Die technische Realisierung der Betätigungseinheit könnte in vorteilhafter Weise durch einen Hexapoden (Steward- oder Flugsimulator-Plattform) gegeben sein. Dieser könnte durch besonders kleine und hoch dynamische Linearmotoren gebildet sein, so dass eine Einheit mit entsprechender Baugröße realisiert werden könnte. Diese könnte problemlos in das handgehaltene Gehäuse integriert werden. Der Hexapod bietet die Möglichkeit, Bewegungen in allen sechs Freiheitsgraden in einem für die Anwendung ausreichend großen Arbeitsbereich auszuführen.

In einer alternativen Ausführungsform könnte die Betätigungseinheit eine Konstruktion aufweisen, die auf der parallelen Anordnung zweier epizyklischer Getriebe beruht. Hierdurch würde mit einer rein rotatorischen Ansteuerung eine Bewegung der Bohrerachse in vier Freiheitsgraden, nämlich Verschiebungen in x- und γ-Richtung sowie Verdrehungen um die x- und γ-Achse - ermöglicht. Die Bewegungen können durch die geringe Anzahl von Bauteilen und die günstige Anordnung sehr präzise, schnell und platzsparend durchgeführt werden.

Der Ausgestaltung des Werkzeugs sind prinzipiell keine Grenzen gesetzt. In medizinischen Anwendungen sind beispielsweise Bohrer denkbar, Fräswerkzeuge zum Ausfräsen von Kavitäten in Knochen oder auch Zangen zum Einsatz im Rahmen einer Biopsie. An dieser Stelle sei noch einmal ganz besonders darauf hingewiesen, dass sich der Einsatz der erfindungsgemäßen Vorrichtung nicht auf medizinische, insbesondere chirurgische Eingriffe beschränkt, sondern dass auch eine präzise Durchführung von schneidenden, spanenden, sägenden oder ähnlichen Bearbeitungsvorgängen im Bereich der automatisierten industriellen Fertigung und/oder im Heimwerkerbereich möglich ist.

Im Hinblick auf eine in Bezug auf die Schwingungen bzw. Wackelbewegungen der Hand des Anwenders ausreichende Beweglichkeit des Werkzeugs könnte vorgesehen sein, dass das Werkzeug in einer speziellen Ausführungsform einen zylindrischen Arbeitsbereich von 40 mm Durchmesser und 40 mm Länge aufweist. Dies entspräche in etwa einer Winkelbeweglichkeit des Werkzeugs in der zx- und zy-Ebene von ± 20°.

Innerhalb des Arbeitsbereichs könnte in einer speziellen Ausführungsform die Genauigkeit der Lageregelung des Werkzeugs so hoch sein, dass sich die Werkzeugspitze stets definiert in einem Würfel von 0,1 mm Kantenlänge befindet. Die Ausrichtung des Werkzeugs sollte dabei nicht mehr als 0,1° von der idealen Richtung abweichen.

Insbesondere für den Einsatz der Vorrichtung im OP-Bereich könnte vorgesehen sein, dass sowohl das Werkzeug als auch das Gehäuse sterilisierbar sind. Hinsichtlich der Bedienungsfreundlichkeit der Vorrichtung könnte vorgesehen sein, dass der Arbeitsprozess, z. B. der Bohrvorgang, zunächst gesperrt ist. Erst wenn das Gerät vom Operateur in die Nähe der geplanten Bohrposition gebracht wurde und die Betätigungseinheit den Bohrer in seiner Winkellage und seiner Position korrekt ausgerichtet hat, wird der Bohrvorgang automatisch freigegeben. Dies könnte beispielsweise durch ein akustisches und/oder ein optisches Signal angezeigt werden.

Während der Operation könnten bestimmte Bearbeitungsparameter automatisch überwacht werden. Bei einem Bohrvorgang ist hier insbesondere an die Vorschubgeschwindigkeit, die Vorschubkraft sowie die Drehzahl des Bohrers zu denken. Nach Erreichen der geplanten Bohrtiefe könnte ein automatisches Abschalten des Bohrers vorgesehen sein.

Das Positionserfassungssystem weist in vorteilhafter Weise eine Abtastrate von mindestens 50 Hz auf. Diese Frequenz ist notwendig, um auch schnelle Bewegungen bzw. höherfrequente Schwingungen, die beim Muskeltremor im Bereich um 12 Hz liegen, erfassen zu können. Das System könnte so ausgelegt sein, dass insgesamt sechs Marker mit einer Abtastrate von 50 Hz erfassbar sind. Hinsichtlich der Genauigkeit in der Positionsbestimmung könnte das Positionserfassungssystem so ausgelegt sein, dass der Fehler kleiner als 0,1 mm ist, je nach Einsatzgebiet sogar kleiner als 0,07 mm.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Darstellung ein Ausführungsbeispiel einer erfin- dungsgemäßen Vorrichtung zur Bearbeitung von Teilen jedweder Art, insbesondere von Knochen, Organen etc. des menschlichen/tierischen Körpers,
- Fig. 2: in einer schematischen Vorder- und Seitenansicht ein Ausführungsbei- spiel eines handgehaltenen Geräts der erfindungsgemäßen Vorrichtung und
- Fig. 3: in einem Blockdiagramm, schematisch, das Zusammenwirken der ein- zelnen Komponenten der erfindungsgemäßen Vorrichtung.

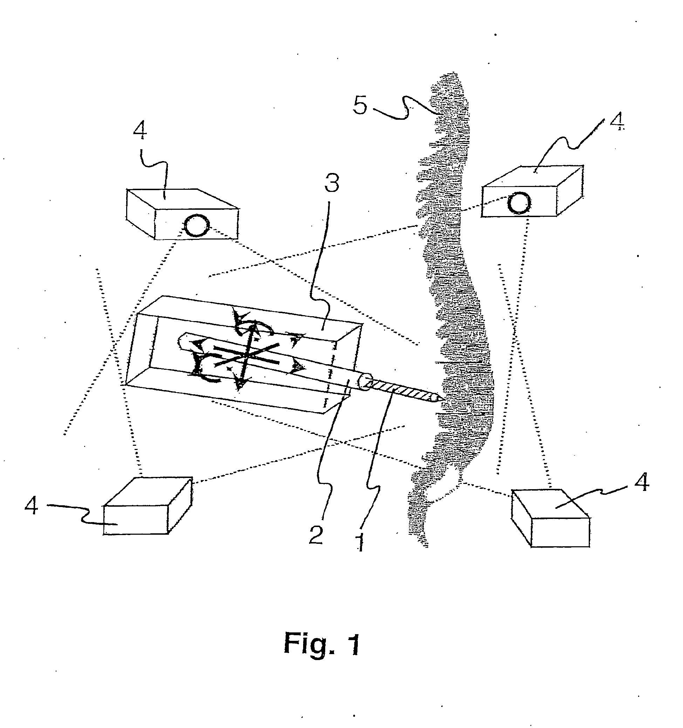

Das in Fig. 1 schematisch dargestellte Ausführungsbeispiel einer erfindungsgemäßen Vorrichtung weist ein als Bohrer ausgebildetes Werkzeug 1 auf, das in eine Werkzeugaufnahme 2 eingespannt ist. Mittels einer Betätigungseinheit, die in Fig. 1 nicht gezeigt ist, kann der Bohrer 1 gegenüber einem Gehäuse 3 in sechs Freiheitsgeraden bewegt werden: Verschiebungen entlang der drei kartesischen Achsen x, y und z sowie Rotationen um die drei Achsen, wobei die Verschiebung in z-Richtung den Bohrervorschub und die Rotation um die z-Achse die Bohrerdrehung bedeutet.

Ein optisches Positionserfassungssystem erfasst die interessierenden Objekte. Die zu detektierenden Objekte sind der Bohrer 1 und/oder das Gehäuse 3 sowie ein zu bearbeitendes Teil, das in Fig. 1 in Form einer Wirbelsäule 5 dargestellt ist. Die Bilder der Kameras 4 werden im PC analysiert. Durch stereoskopische Rückprojektion kann aus den Kamerabildern die Lage der Objekte im Raum bestimmt werden.

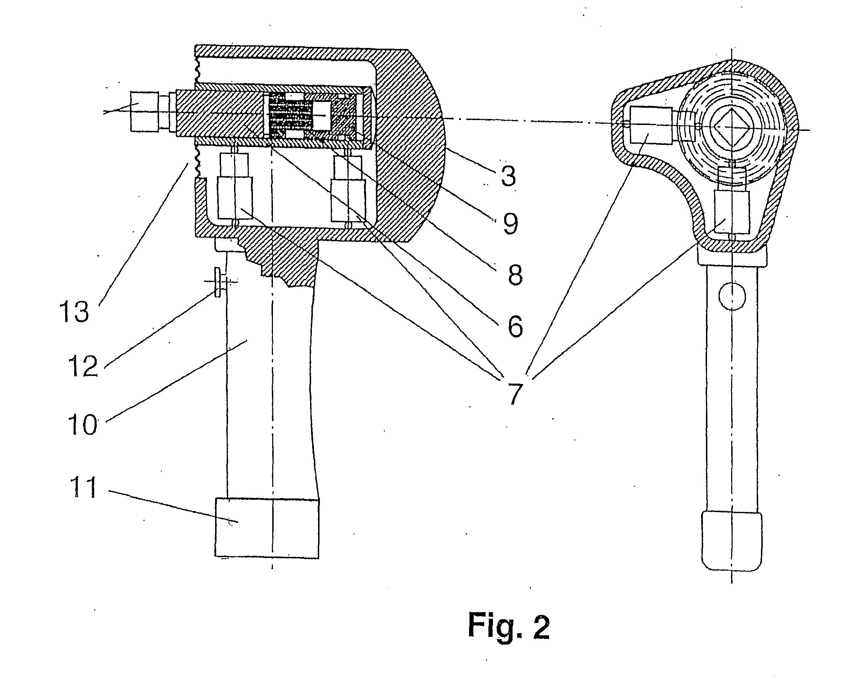

In Fig. 2 ist in einer schematischen Seiten- und Vorderansicht ein Ausführungsbeispiel eines handgehaltenen Geräts der erfindungsgemäßen Vorrichtung dargestellt. Innerhalb eines Gehäuses 3 ist ein Träger 6 angeordnet, dessen vorderes Ende eine Werkzeugaufnahme 2 aufweist, in der unterschiedliche Werkzeuge auswechselbar eingespannt werden können. Der Träger 6 umfasst eine elektrisch gesteuerte Drehachse zum Antrieb des Werkzeugs, z. B. eines Bohrers. Zwischen dem Träger 6 und dem Gehäuse 3 wirken Linear-Aktoren 7, die den Träger 6 und damit den Bohrer in eine - innerhalb eines vorgegebenen Arbeitsbereichs - beliebige Relativlage gegenüber dem Gehäuse 3 verbringen können. Der Vorschub des Werkzeugs wird über eine auf der Bohrerachse hinter dem Träger 6 angeordnete elektrisch gesteuerte z-Achse 8 erzeugt. Zusätzlich ist eine pneumatische Schlageinheit 9 vorgesehen, so dass das Gerät zur Bearbeitung von Teilen unterschiedlicher Beschaffenheit, insbesondere unterschiedlicher Härte flexibel eingesetzt werden kann.

Das Gehäuse 3 ist mit einem Handgriff 10 versehen, der an seinem dem Gehäuse 3 abgewandten Ende zum Ausgleich des Gewichts der Betätigungseinheit ein Gegengewicht 11 aufweist. An dem Handgriff 10 ist ein Betätigungsschalter 12 angeordnet, mit dem der Anwender den Arbeitsvorgang auch manuell unterbrechen bzw. fortsetzen kann. Die Gehäuseöffnung, durch die der Träger 6 austritt, ist zum Schutz der Betätigungseinheit und der inneren Sensorik vor Beschädigungen und Verschmutzungen mit einer Abdichtungsmembrane 13 verschlossen.

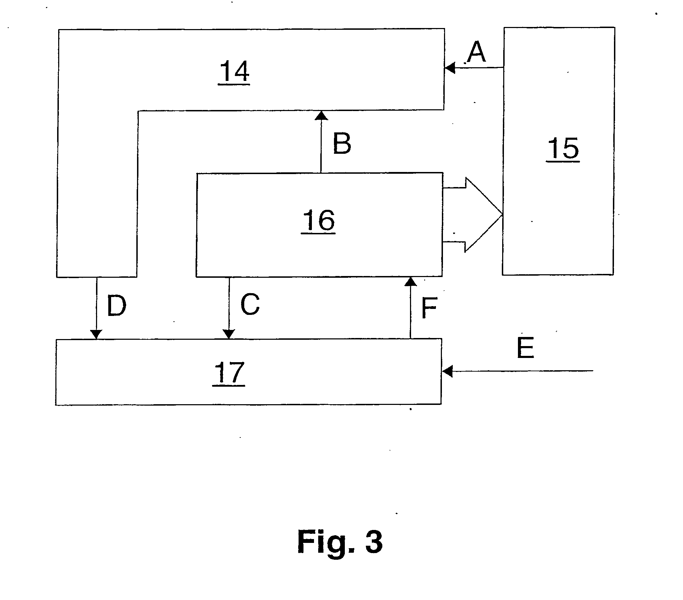

Fig. 3 verdeutlicht in einer schematischen Darstellung das Zusammenspiel der einzelnen Komponenten einer erfindungsgemäßen Vorrichtung. Ein Positionserfassungssystem 14 detektiert mit einer bestimmten Abtastrate die IST-Koordinaten A eines Patienten 15 oder ganz allgemein eines zu bearbeitenden Teils und die IST-Koordinaten B des Handgeräts 16 oder genauer gesagt die IST-Koordinate des Werkzeugs. Hierzu sind die entsprechenden zu detektierenden Stellen z. B. im Sinne der o. g. Anmeldung DE 102 25 077.4 gekennzeichnet. Die IST-Position des Werkzeugs kann dabei entweder direkt bestimmt werden, oder es wird über das Positionserfassungssystem 14 die IST-Position des Gehäuses bestimmt und mit einem zusätzlichen, in der Fig. 2 nicht dargestellten Sensor die relative Lage C des Bohrers im Koordinatensystem des Gehäuses gemessen.

Die relative Lage C sowie die relative Lage D des Gehäuses 3 im patientenfesten Koordinatensystem werden zur Auswertung an eine adaptive und schnelle Reglereinheit 17 weitergegeben. Diese vergleicht die IST-Positionsdaten mit den SOLL-Positionsdaten E aus der (präoperativen) OP-Planung und generiert eine Steuergröße F für eine ebenfalls nicht dargestellte Betätigungseinheit. Die Betätigungseinheit umfasst Aktoren und verbringt das Werkzeug unabhängig von den Bewegungen des Operateurs und des Patienten 15 in die gewünschte Position.

Hinsichtlich weiterer vorteilhafter Ausgestaltungen und Weiterbildungen der erfindungsgemäßen Lehre wird einerseits auf den allgemeinen Teil der Beschreibung und andererseits auf die beigefügten Patentansprüche verwiesen.

Abschließend sei ganz besonders hervorgehoben, dass das zuvor rein willkürlich gewählte Ausführungsbeispiel lediglich zur Erörterung der erfindungsgemäßen Lehre dient, diese jedoch nicht auf das Ausführungsbeispiel einschränkt.

## Patentansprüche

1. Vorrichtung zur manuellen Bearbeitung von Teilen jedweder Art, insbesondere von Knochen, Organen etc. des menschlichen/tierischen Körpers, mit einem zur Handhaltung vorgesehenen Gehäuse (3), einem dem Gehäuse (3) zugeordneten Werkzeug (1) und einer eine Relativbewegung zwischen dem Gehäuse (3) und dem Werkzeug (1) bewirkenden Betätigungseinheit,
**dadurch gekennzeichnet, dass** die Position des Werkzeugs (1) detektierbar und die Position des zu bearbeitenden Teils detektierbar und/oder vorgebbar ist, wobei die Betätigungseinheit so ansteuerbar ist, dass sie das Werkzeug (1) unter Berücksichtigung von Bewegungen des handgehaltenen Gehäuses (3) sowie des zu bearbeitenden Teils innerhalb eines vorgegebenen Arbeitsbereichs in eine vorgebbare Relativlage gegenüber dem zu bearbeitenden Teil verbringt, wobei eine Reglereinheit (17) zur Auswertung der Positionsdaten und zur Generierung der Steuergrößen für die Betätigungseinheit vorgesehen ist.

2. Vorrichtung nach Anspruch 1, **dadurch gekennzeichnet, dass** es sich bei der Reglereinheit (17) um eine adaptive Reglereinheit (17) handelt.

3. Vorrichtung nach Anspruch 1 oder 2, **dadurch gekennzeichnet, dass** die Detektion der Positionen von Werkzeug (1) und zu bearbeitendem Teil und das Ansteuern der Betätigungseinheit kontinuierlich oder repetitiv durchführbar sind.

4. Vorrichtung nach einem der Ansprüche 1 bis 3, **dadurch gekennzeichnet, dass** das Werkzeug (1) gegenüber dem Gehäuse (3) in mehreren, vorzugsweise in sechs Freiheitsgraden bewegbar ist.

5. Vorrichtung nach einem der Ansprüche 1 bis 4, **dadurch gekennzeichnet, dass** zur Detektion der Positionen von Werkzeug (1) und zu bearbeitendem Teil ein als externes optisches und/oder akustisches und/oder magnetisches und/oder mechanisches und/oder radioaktives System ausgebildetes Positionserfassungssystem (14) vorgesehen ist.

6. Vorrichtung nach einem der Ansprüche 1 bis 5, **dadurch gekennzeichnet, dass** dem Werkzeug (1) und dem zu bearbeitenden Teil und/oder dem Gehäuse (3) jeweils mindestens ein detektierbarer Marker zugeordnet ist.

7. Vorrichtung nach einem der Ansprüche 1 bis 6, **dadurch gekennzeichnet, dass** dem Gehäuse (3) und/oder dem Werkzeug (1) mittelbar oder unmittelbar mindestens ein Sensor zur Bestimmung der Relativlage zwischen dem Gehäuse (3) und dem Werkzeug (1) zugeordnet ist, wobei der mindestens eine Sensor ein mechanischer Weggeber, ein induktiver Weggeber, und/oder ein Winkelsensor sein kann.

8. Vorrichtung nach einem der Ansprüche 1 bis 7, **dadurch gekennzeichnet, dass** die Betätigungseinheit Aktoren (7) umfasst, wobei die Aktoren (7) innerhalb des Arbeitsbereichs in jeder Raumrichtung Kräfte von bis zu 20 N aufbringen.

9. Vorrichtung nach einem der Ansprüche 1 bis 8, **dadurch gekennzeichnet, dass** die Betätigungseinheit als Hexapod-Roboter ausgeführt ist.

10. Vorrichtung nach einem der Ansprüche 1 bis 9, **dadurch gekennzeichnet, dass** das Werkzeug (1) ein Bohrer, eine Fräse, eine Zange, eine Nadel oder ähnliches ist und/oder dass das Werkzeug (1) einen zylindrischen Arbeitsbereich von 40 mm Durchmesser und 40 mm Länge aufweist.

11. Vorrichtung nach einem der Ansprüche 1 bis 10, **dadurch gekennzeichnet, dass** die Freigabe eines Bohrvorgangs automatisch erfolgt, wobei die Freigabe nach Erreichen der vorgegebene Relativlage zwischen Werkzeug (1) und dem zu bearbeitenden Teil erfolgen kann und/oder wobei ein akustisches und/oder optisches Signal die Freigabe des Bohrvorgangs anzeigen kann.

12. Vorrichtung nach einem der Ansprüche 1 bis 11, **dadurch gekennzeichnet, dass** Vorschubgeschwindigkeit, -kraft, und Drehzahl während der Bearbeitung automatisch überwacht werden.

## Claims

1. A device for manual processing of parts of any kind, in particular bones, organs etc. of the human/animal body, having a housing (3) provided for manipulation, a tool (1) associated with the housing (3) and an operating unit effecting a relative movement between the housing (3) and the tool (1), **characterised in that** the position of the tool (1) is detectable and the position of the part to be processed is detectable and/or predeterminable, wherein the operating unit is arranged to be driven in such a way that, allowing for movements of the hand-held housing (3) as well as of the part to be processed, it brings the tool (1) within a predetermined working area into a predeterminable relative position with respect to the part to be processed, wherein a controller unit (17) is provided for evaluating the position data and for generating the control variables for the operating unit.

2. A device according to claim 1, **characterised in that** the controller unit (17) is an adaptive controller unit (17).

3. A device according to claim 1 or 2, **characterised in that** the detection of the positions of tool (1) and part to be processed and the driving of the operating unit are carried out continuously or repeatedly.

4. A device according to any one of claims 1 to 3, **characterised in that** the tool (1) is movable with respect to the housing (3) in a plurality, preferably in six, degrees of freedom.

5. A device according to any one of claims 1 to 4, **characterised in that** for detection of the positions of tool (1) and part to be processed, a position finding system (14) in the form of an external optical and/or acoustic and/or magnetic and/or mechanical and/or radioactive system is provided.

6. A device according to any one of claims 1 to 5, **characterised in that** at least one detectable marker is associated in each case with the tool (1) and with the part to be processed and/or with the housing (30).

7. A device according to any one of claims 1 to 6, **characterised in that** at least one sensor for determining the relative position between the housing (3) and the tool (1) is directly or indirectly associated with the housing (3) and/or with the tool (1), wherein the at least one sensor can be a mechanical displacement sensor, an inductive displacement sensor and/or an angle sensor.

8. A device according to any one of claims 1 to 7, **characterised in that** the operating unit comprises actuators (7), the actuators (7) applying forces of up to 20 N within the working area in each direction in space.

9. A device according to any one of claims 1 to 8, **characterised in that** the operating unit is in the form of a hexapod robot.

10. A device according to any one of claims 1 to 9, **characterised in that** the tool (1) is a drill bit, a milling cutter, pincers, a needle or similar and/or the tool (1) has a cylindrical working area of 40 mm diameter and 40 mm length.

11. A device according to any one of claims 1 to 10, **characterised in that** the enabling of a drilling procedure is effected automatically, wherein the enabling can be effected after the predetermined relative position between tool (1) and the part to be processed has been reached and/or wherein an acoustic and/or optical signal can indicate the enabling of the drilling procedure.

12. A device according to any one of claims 1 to 11, **characterised in that** feed rate, feed pressure and rotation speed are automatically monitored during the processing.

## Revendications

1. Dispositif pour le travail manuel de parties du genre utiles, notamment des os, des organes etc. du corps humain ou animal, avec un boîtier (3) prévu pour la manipulation, un outil (1) affecté au boîtier (4) et une unité d'actionnement provoquant un mouvement relatif entre le boîtier (3) et l'outil (1),
**caractérisé en ce que** la position de l'outil (1) peut être détectée et la position de la partie à travailler peut être détectée et/ou prédéterminée, l'unité d'actionnement étant commandée de telle façon qu'elle déplace l'outil (1) en tenant compte des mouvements du boîtier (3) tenu à la main ainsi que de la partie à travailler dans une zone de travail prédéterminée , dans une position relative par rapport à la partie à travailler, une unité de réglage (17) étant prévue pour l'évaluation des données de position et pour générer les grandeurs de commande pour l'unité d'actionnement.

2. Dispositif selon la revendication 1, **caractérisé en ce que** l'unité de réglage (17) est une unité de réglage (17) adaptative.

3. Dispositif selon la revendication 1 ou 2, **caractérisé en ce que** la détection des positions de l'outil (1) et de la pièce à travailler et la commande de l'unité d'actionnement peuvent être effectuées de façon répétitive ou continue.

4. Dispositif selon l'une des revendications 1 à 3, **caractérisé en ce que** l'outil (1) est mobile par rapport au boîtier (3) dans plusieurs, de préférence six degrés de liberté.

5. Dispositif selon l'une des revendications 1 à 4, **caractérisé en ce que** pour détecter les positions de l'outil (1) et de la partie à travailler, il est prévu un dispositif détecteur de position constitué par un système optique externe et /ou un système acoustique et/ou magnétique et/ou mécanique et/ou radioactif (14).

6. Dispositif selon l'une des revendications 1 à 5, **caractérisé en ce qu'**au moins un marquage détectable est individuellement attribué à l'outil (1) et à la partie à travailler et/ou au boîtier (3).

7. Dispositif selon l'une des revendications 1 à 6, **caractérisé en ce qu'**au moins un capteur pour déterminer la position relative entre le boîtier (3) et l'outil (1) est attribué directement ou indirectement au boîtier (3) et/ou à l'outil (1), le au moins un capteur pouvant être un indicateur mécanique de trajet un indicateur inductif de trajet et/ou un capteur angulaire.

8. Dispositif selon l'une des revendications 1 à 7, **caractérisé en ce que** l'unité d'actionnement comporte des actionneurs (7), ces actionneurs (7) appliquant dans la zone de travail, dans chaque direction de l'espace, des forces allant jusqu'à 20 N.

9. l'unité d'actionnement est constitue par un robot hexapode.

10. Dispositif selon l'une des revendications 1 à 9, **caractérisé en ce que** l'outil (1) est un foret, une fraise, une pince, une aiguille ou analogue et/ou que l'outil (1) présente une zone de travail cylindrique de 40 mm de diamètre et 40 mm de hauteur.

11. Dispositif selon l'une des revendications 1 à 10, **caractérisé en ce que** le déclenchement d'un processus de perçage se fait automatiquement, le déclenchement pouvant se produire après atteinte de la position relative prédéterminée entre l'outil (1) et la partie à travailler et/ou un signal acoustique et/ou optique pouvant indiquer le déclenchement du processus de perçage.

12. Dispositif selon l'une des revendications 1 à 11, **caractérisé en ce que** la vitesse et la force d'avance et la vitesse de rotation sont automatiquement contrôlées pendant le travail.
